# EUROPEAN PATENT SPECIFICATION

(11) **EP 2 566 491 B1**
(45) Date of publication and mention of the grant of the patent: **08.07.2015**
(21) Application number: 11724119.0
(22) Date of filing: 09.05.2011
(51) Int. Cl.: A61K 35/76, C12N 7/00

(54) **MODIFIED RODENT PARVOVIRUS CAPABLE OF PROPAGATING AND SPREADING THROUGH HUMAN GLIOMAS**
MODIFIZIERTES NAGER-PARVOVIRUS, DAS SICH DURCH MENSCHLICHE GLIOME VERMEHREN UND AUSBREITEN KANN
PARVOVIRUS DE RONGEUR MODIFIÉ CAPABLE DE SE PROPAGER ET DE S'ÉTALER À TRAVERS LE GLIOME HUMAIN

(30) Priority: 07.05.2010 EP 10004897
(43) Date of publication of application: 13.03.2013
(73) Proprietor: Deutsches Krebsforschungszentrum, 69120 Heidelberg (DE)
(72) Inventor: NÜESCH, Jürg, 69124 Heidelberg (DE); THOMAS, Nadja, 35390 Gießen (DE); PLOTZKY, Claudia, 68549 Ilvesheim (DE); ROMMELAERE, Jean, 69118 Heidelberg (DE)
(74) Representative: Schüssler, Andrea
(86) International application number: PCT/EP2011/002306
(87) International publication number: WO 2011/138053

(56) References cited:
- US-A1- 2004 220 124
- HERRERO Y CALLE MARTA ET AL: "Parvovirus H-1 infection of human glioma cells leads to complete viral replication and efficient cell killing.", INTERNATIONAL JOURNAL OF CANCER, vol. 109, no. 1, 10 March 2004 (2004-03-10), pages 76-84, XP002614412, ISSN: 0020-7136
- BÄR SÉVERINE ET AL: "Vesicular egress of non-enveloped lytic parvoviruses depends on gelsolin functioning.", PLOS PATHOGENS 2008 LNKD- PUBMED:18704167, vol. 4, no. 8, 2008, page E1000126, XP002614413, ISSN: 1553-7374

## Description

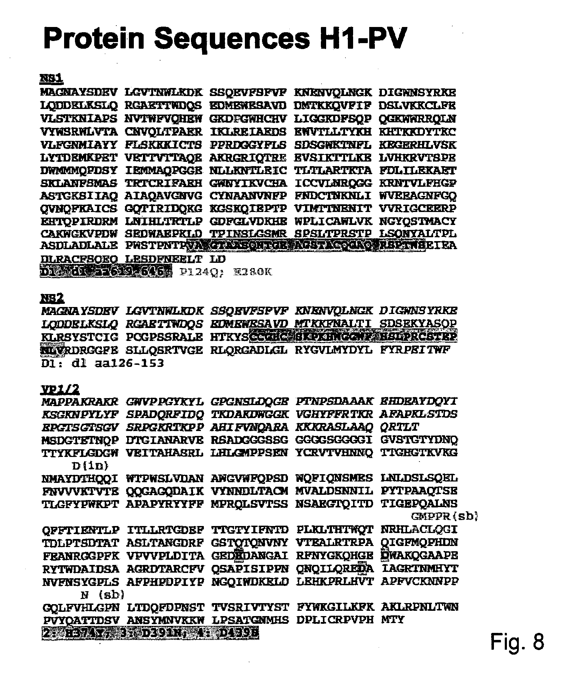

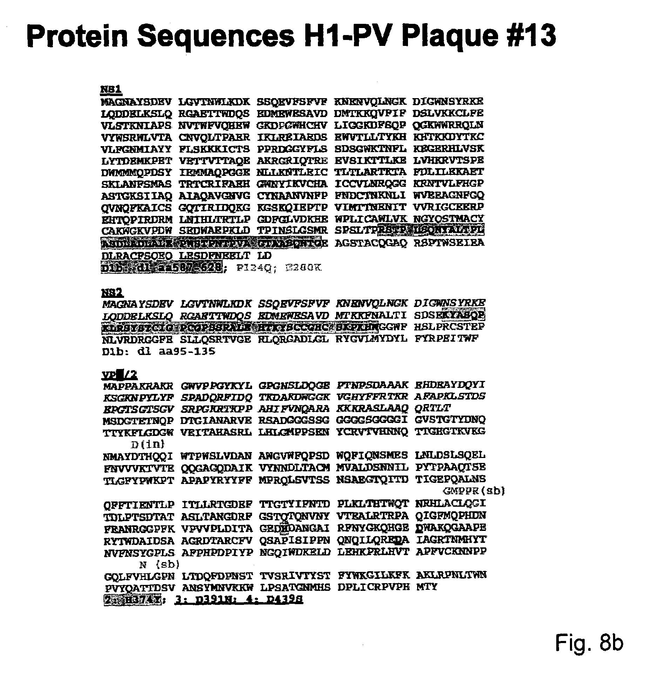

The present invention relates to the embodiments characterized in the claims. Thus, it relates to a rat parvovirus H-1 variant capable of propagating and spreading through human tumour cells, characterized in that the viral polypeptides comprise the amino acid sequences shown in Figure 8B and which have a deletion from position 587 to 629 in the C-terminus of NS1 and the substitutions P124Q and E280K in NS1, a deletion from position 95 to 136 in the middle exon of NS2 and the amino acid substitution H374Y in VP1/VP2.

Oncolytic viruses such as rodent parvoviruses represent novel tools for cancer treatment. Besides specifically killing cancer cells (oncolysis), these agents also provide danger signals prompting the immune system to eliminate virus-infected tumours. As a consequence of oncolytic events, the innate and adaptive immune systems gain access to tumour antigens, which results in cross-priming and vaccination effects. Rodent parvoviruses are single-stranded DNA viruses possessing intrinsic oncolytic activity, i.e. they preferentially replicate in and kill cancer cells of both murine and human origin.

Glioblastoma is a devastating disease with only limited treatment possibilities. Evidently, the prognosis for patients requires new therapies. The replication competent oncolytic viruses discussed above are considered promising since they are able to spread through malignant tissues and induce antitumor immune-responses. Among them, rodent parvoviruses appear to be excellent candidates, due to their natural oncotropism, their capacity to infect human cells, the specific toxicity for neoplastic cells, and the low pathogenicity for humans. In a rat model, H1-PV was able to cause a complete regression of established gliomas without any relaps and they proved able to target various human gliomas, thereby efficiently killing these cells irrespective of their acquired resistance towards known death inducers. Although, H1-PV proved able to infect and efficiently kill most of the human glioblastoma cell lines tested so far, the majority of these cells resisted efficient virus replication, progeny particle production and spreading. This failure to propagate and spread through human tumor tissue could have major impact for the treatment efficacy of human gliomas *in vivo,* not only because cell killing is limited to a single hit event reaching only a limited proportion of the tumor cells, but also due to the lack of an active intracellular transport of progeny virions to the cell surface.

Therefore, it is the object of the present invention to overcame this draw back of the prior art strains, i.e., to provide parvoviruses that are capable to propagate and spread through human tumor tissue.

According to the invention this is achieved by the subject matters defined in the claims. Initial experiments of the inventors have shown that progeny PV virions are shuttled from the nucleus to the cell periphery through vesicles prior to cell lysis where they are released into the medium supernatant. Moreover, this vesicular egress is associated with multiple intracellular proteins (including potential tumor antigens) which are carried as "co-cargo" to the cell surface and might contribute to the host anti-tumor immune stimulation. To mend the defects of the original H1-PV isolate (pSR19) in virus propagation and spreading in human glioblastoma, the inventors aimed to obtain propagation competent H1-PV variants through serial passaging in semi-permissive patient-derived human glioblastoma cell lines, i.e., to circumvent potential bottle-necks during the infection process (uncoating, delivery of the viral genome to the nucleus, double-strand conversion), infectious clone DNA of H1-PV (pSR-19) was transfected into different human glioblastoma cell lines (e.g. NCH149, NCH82, NCH89) and this replication competent DNA was passaged until cytopathic effects became visible. Additional viral passages (> 15) were made combining cell associated progeny virions obtained through repeated freezing and thawing in vTE pH 8.7 with medium released viruses through infection. A pool of propagation competent viruses was then analyzed after 25 passages on NCH149 cells presenting efficient viral DNA amplification, generation of progeny virions and spreading through a variety of human glioblastoma cultures. From this original pool, individual virus clones were isolated through plaque-purification on NB 324K cells, amplified again on NCH149 cells, cloned and sequenced.

The genetic analysis of all H1-PV variants has revealed a deletion of 84 nts affecting 28 amino acids of the coding region of NS1 (C-terminus) and NS2 and a single cytosine to thymidine transition at position 3913 changing His374 to Tyr in VP2. Two additional guanidine to adenosine substitutions in the VP2 region (3964 and 4108) leading to a change from Asp391 to Asn and Asp439 to Ser have only been assigned to two isolates. A few changes in the 3' non-coding regions remained of various prevalence. Apparently, the region approximately between nts 2000 to 2200 comprises a hot-spot of variability allowing H1-PV through modulation of NS1/NS2 function to adapt to the host environment. Therefore, it is possible to modify actively the host range of H1-PV, but also the other closely related rat PV strains, H3 and TVX through insertion/deletions within this region.

The newly generated H1-PV variants are able to propagate and spread through human glioblastoma cell(line)s. This allows an increased infection/destruction of already established human tumors and in consequence it is expected to give rise to a better anti-tumor immune stimulation. In agreement with the inventors' previous findings of parvovirus vesicular egress, it is reasonable to assume that the co-transport of intracellular proteins to the surface might serve to unmask tumor cells for a host immune response. This co-cargo effect is not present in absence of progeny virion formation using the original H1-PV isolate (pSR-19).

### Summary of the present Invention

With the adaptation process (passaging) in semi-permissive cells as outlined in the examples an H1-PV variant proficient for propagation in many other human glioblastoma cell lines could be generated. The outlined procedure can be expanded to other human tumor cell(line)s including tumor stem cells.

Adaptation of the rat H1-PV on human glioblastoma cells led to a deletion in the C-terminus of H1 NS1/middle exon of NS2. This region represents a hot spot area for variation determining H1-PV host range and with additional adapatations/analyses of other so far uncharacterized rodent PV isolates additional variations can be found in this area.

Besides generating an H1-PV variant that is potentially able to spread from cell to cell in human glioblastoma tumor tissue and therefore has a higher potential for tumor cell killing (as indicated by the fitness assays), progeny particles of this isolate are actively transported to the cell periphery through vesicles. Besides newly synthesized virions, multiple cellular proteins being associated with such vesicles were found after infection which were not present in non-infected human glioblastoma cells. This co-cargo could serve to unmask tumor cells to make them available for the host immune system, thereby contributing for a virus-induced anti-tumor immune response.

### Brief description of the drawings

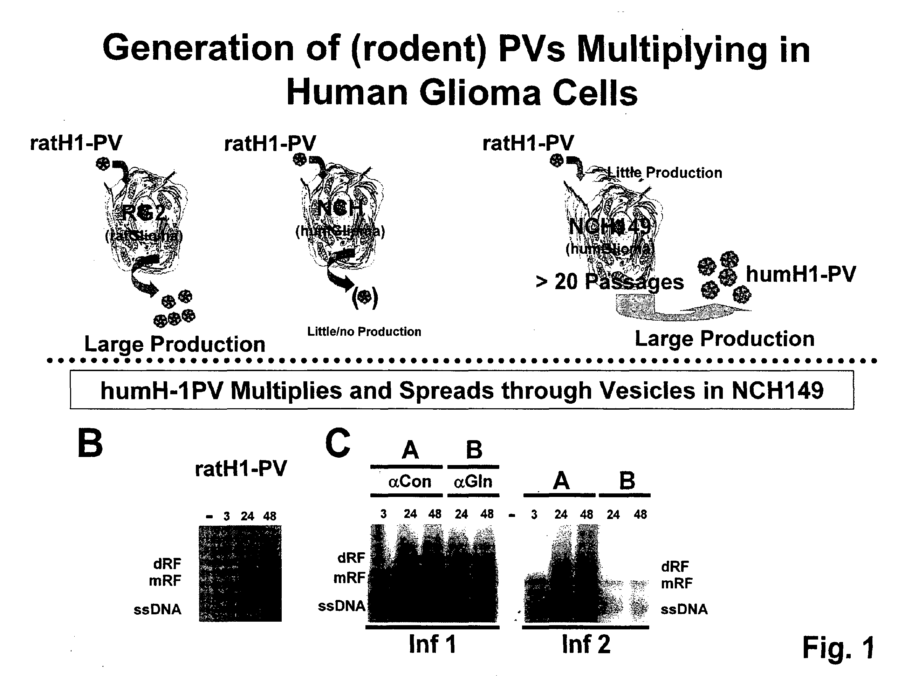

### Figure 1: (Top) Schematical presentation of the adaptation approach

Rat parvovirus H1-PV readily propagates in transformed rat cells such as rat glioma RG-2 (left), but is limited to produce and spread in human glioblastoma cell lines. To overcome these limitations, serial passages were performed in semi-permissive human glioblastoma allowing mutations and amplification of permissive H1-PV variants to occur. Propagation competent variants were obtained/analyzed after 20 consecutive passages in NCH149 cells.

### (B, C) Determination of viral DNA amplification in NCH149

NCH149 cells were infected with 10 pfu/cell H1-PV and the presence of replication intermediates was measured by Southern blotting after indicated times post infection.
(B) Infection with rat H1-PV (pSR19).
(C) Primary and secondary DNA amplification with human glioma adapted H1-PV, determination of gelsolin functioning for vesicular egress and spreading. Inf 1, primary infection, Inf 2, supernatants from Inf 1 were used to infect naive NCH149 cells. The generation of viral replication intermediates was measured by Southern blotting. αCon, control serum; αGln, neutralizing antiserum raised against gelsolin; dRF, dimeric replication intermediate; mRF, monomeric replication intermediate; ssDNA, progeny virus particles = single-stranded virus genomes.

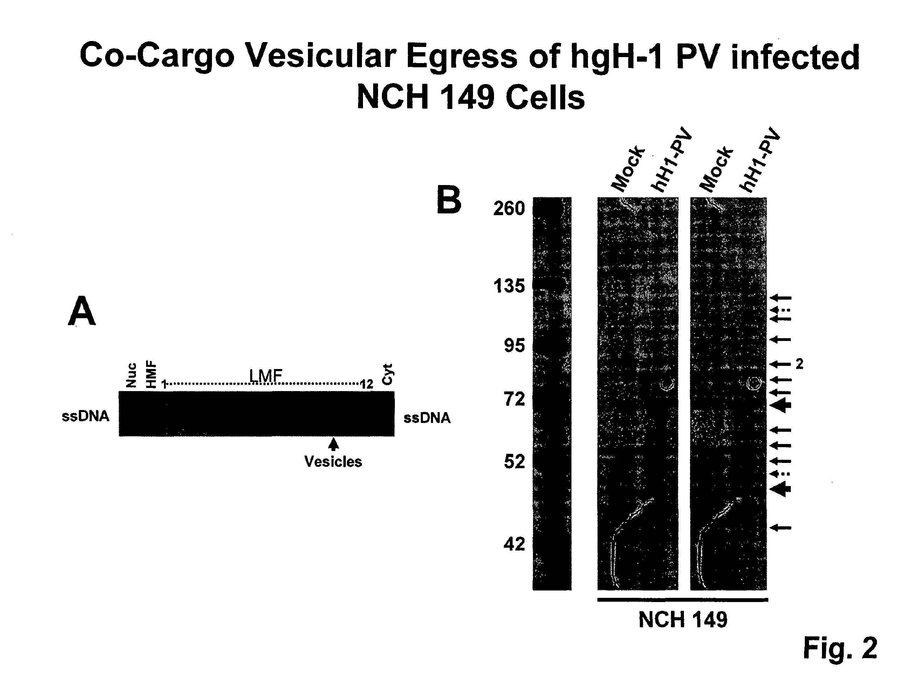

### Figure 2: Determination of H1-PV vesicular egress and identification of "co-cargo"

**(A)** NCH149 cells were infected with 10 pfu/cell human glioma adapted H1-PV and fractionated for nuclei (Nuc), heavy mitochondrial fraction (HMF), and light mitochondrial fraction (LMF), containing cellular vesicles. To determine the presence of progeny virions in the vesicular fraction, LMF components were further separated by iodexanol gradients and the presence of virion DNA was measured by Southern blotting as compared to the presence of vesicular markers (determined by Western blotting).
**(B)** Determination of vesicular proteins in hgH1-PV and mock infected NCH149 cells. Vesicles were purified as described under (A) and analyzed for their protein content by SDS-PAGE and silver staining. Proteins overrepresented in hgH1-PV infected cellular vesicles are indicated by arrows.

### Figure 3:

### (A) Determination of viral protein expression after infection of NCH149 cells with rat (pSR19) versus human glioma adapted H1-PV

NCH149 cells were infected with 10 pfu/cell and harvested at indicated times post infection. Accumulation of viral proteins were determined by Western blotting. Encircles (red) the large heterogeneity of NS1 produced after hgH1-PV infection and the larger accumulation of NS2 and NS3 (blue).

### (B) RG-2 and NCH149 cells were infected with the indicated virus preparations at a multiplicity of 10, and metabolically labelled with ³²P-orthophosphate at 24 h p.i. for four hours

Cells were harvested, NS1 purified by immunoprecipitations and SDS-PAGE and the tryptic phosphopeptide pattern was determined by two-dimensional electrophoresis/chromatography. The phosphopeptide pattern of NS1 produced by rat H1-PV (pSR-19) in rat glioma (left), NCH149 (middle), as well as hgH1-PV in NCH149 cells (right) are shown. Encircled: phosphorylations known to drive viral DNA amplification.

### Figure 4:

### (A) Multiplication of input virus after infection of various target cell lines

Indicated cell lines were infected at a multiplicity 10, washed and harvested 4 (Input virus) 24 and 48 h post infection. Cell and medium associated virus was determined by plaque assay. The average amplification 24 and 48 h post infection is expressed over input (4 h) virus titer.

### (B) Virus fitness assay

Target cell lines were seeded in 24 well dishes, infected at indicated multiplicities with the appropriate virus preparations, and living cells were fixed and stained with crystal violet 6 days post infection.

### Figure 5:

### Top: Genome organization of H1-PV with indication of variation found after adaptation in human glioma

### Middle section: PCR-fragments generated from plaque purified virus preparations

### Bottom: Genetic determination of plaque purified hgH1-PV variants

Cloned PCR fragments are indicated by black lines, sequenced material is indicated by dotted squares and the presence of the individual variations are indicated in green.

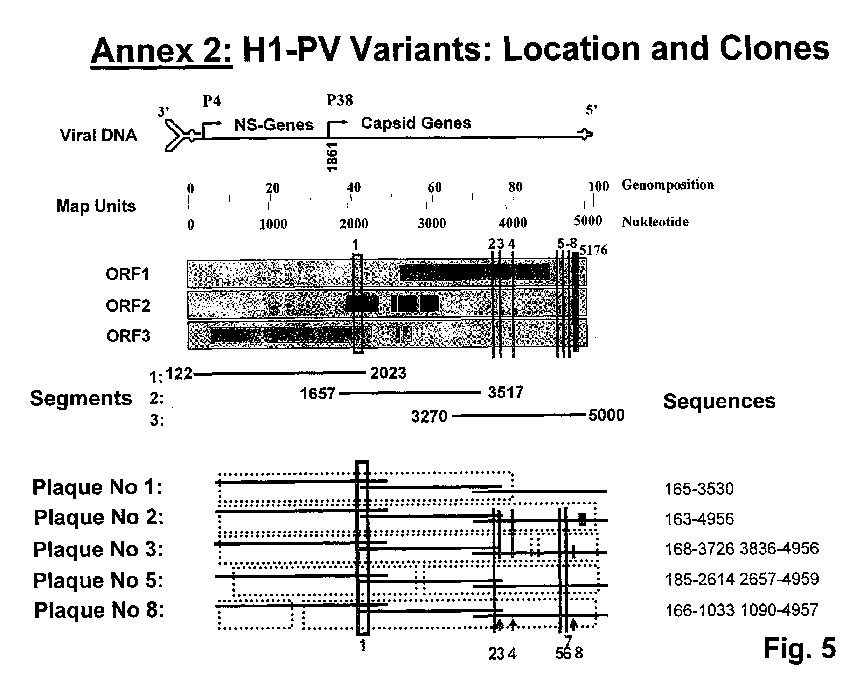

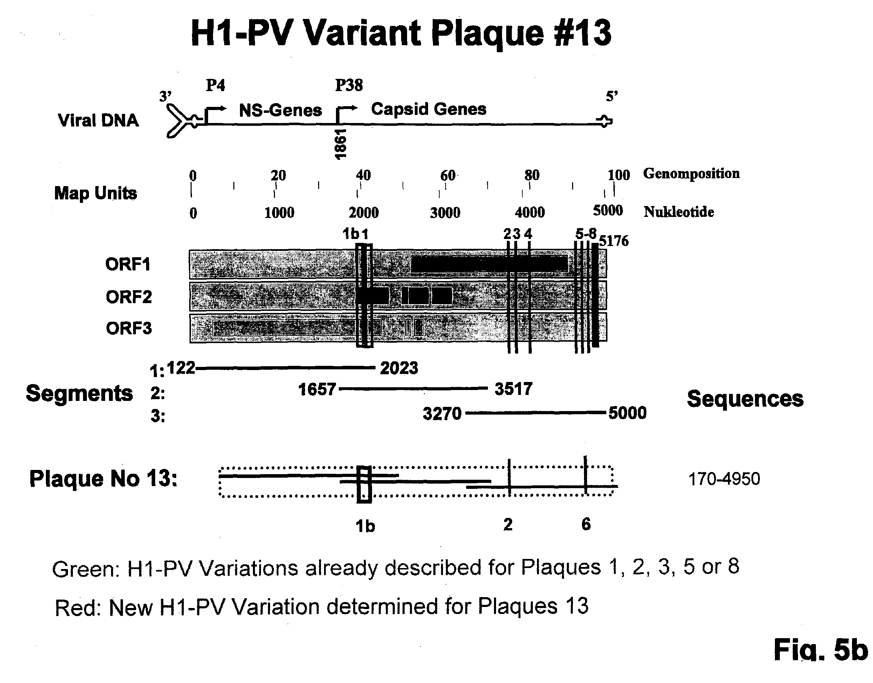

### Figure 5b: H1-PV variant plaque #13:

After the initial characterization of five plaques (1,2,3,5, and 8) an additional plaque derived from the same adaptation experiment was cloned and sequenced (plaque #13). This additional variant was recognized by a different deletion in the C-terminal part of NS1/middle exon of NS2 together with mutations 2 and 6 previously found in the first round of characterizations (plaques 1 through 8).

### Figure 6: Detailed characterization of all genetic variations found by sequencing of individual plaques

### Figure 6b: Detailed characterization of the additional deletion in the coding region of NS1/NS2 found by sequencing of plaque #13.

### Figure 7: Virus fitness assay of virus stocks derived from individual plaque isolates

### Figure 8: Changes in the H1-PV Protein sequences as deduced from the nucleotide changes obtained by DNA-sequencing and summarized in Fig. 6.

Protein sequences corresponding to the H1-PV nucleotide sequence published under NC_001358. The three exons of NS2p as deduced from the transcription pattern of Qiu et al., (2006) are indicated in *italics* (common Nterminus with NS1 = exon1)-regular (middle exon)- italics(C-terminal exon [unique for each NS2 isoform). Amended sequencing errors are indicated in light gray (these differences to the published sequence were found in all our isolates as well). Variations created through passaging in NCH149 cells are indicated in bold and marked with light-grey. VP1-specific sequences are denoted in *italics.*

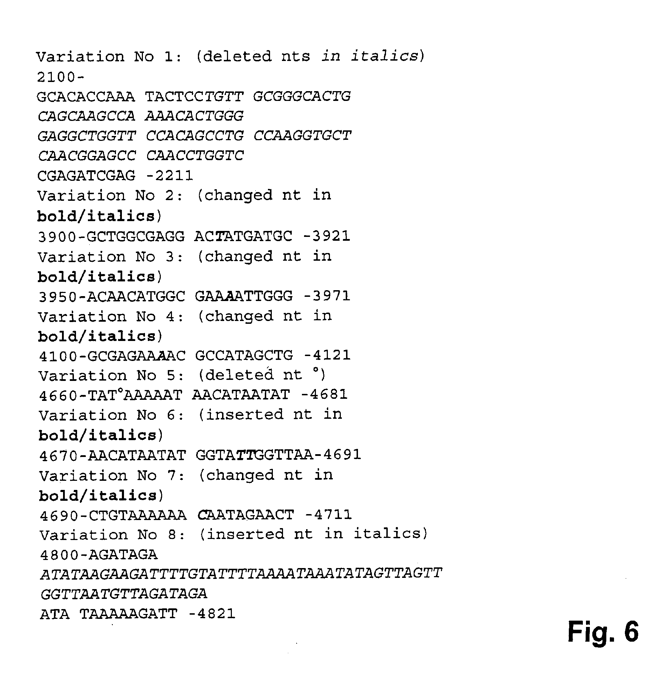

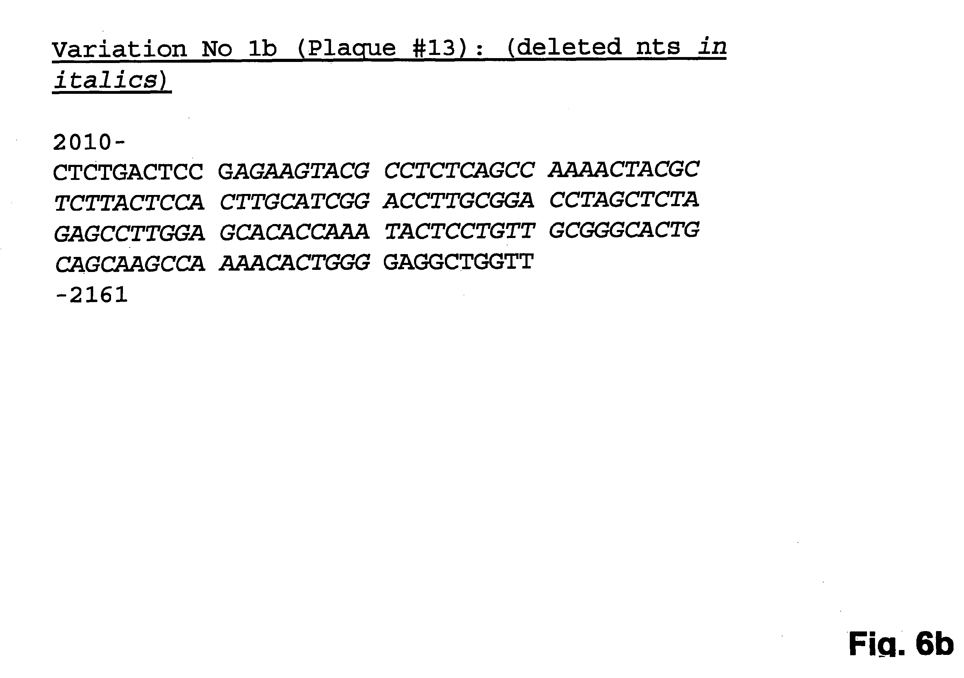

### Figure 8b: Changes in the H1-PV protein sequences in plaque #13 as deduced from nucleotide changes obtained by DNA-sequencing and summarized in Fig. 6b.

Protein sequences corresponding to the H1-PV nucleotide sequence published under NC_001358. The three exons of NS2p as deduced from the transcription pattern of Qiu et al., (2006) are indicated in *italics* (common Nterminus with NS1 = exon1)-regular (middle exon) - *italics* (C-terminal exon [unique for each NS2 isoform). Amended sequencing errors are indicated in light gray (these differences to the published sequence were found in all our isolates as well). Variations created through passaging in NCH149 cells are indicated in bold. VP1-specific sequences are denoted in italics. Coding changes of the deletion D1b and VP1/2 mut4 of Plaque #13 are given in bold and marked with light-gray.

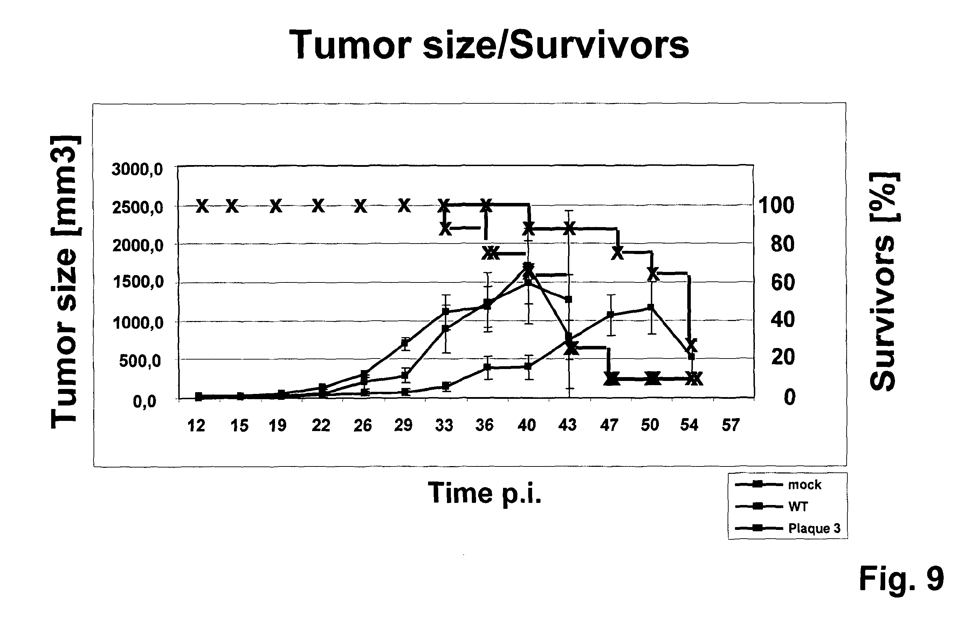

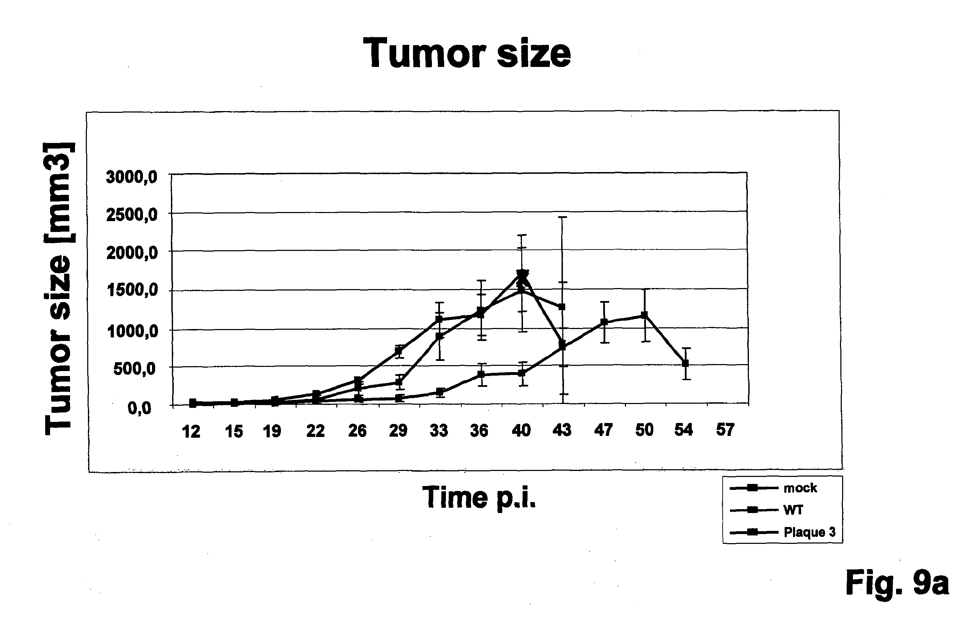

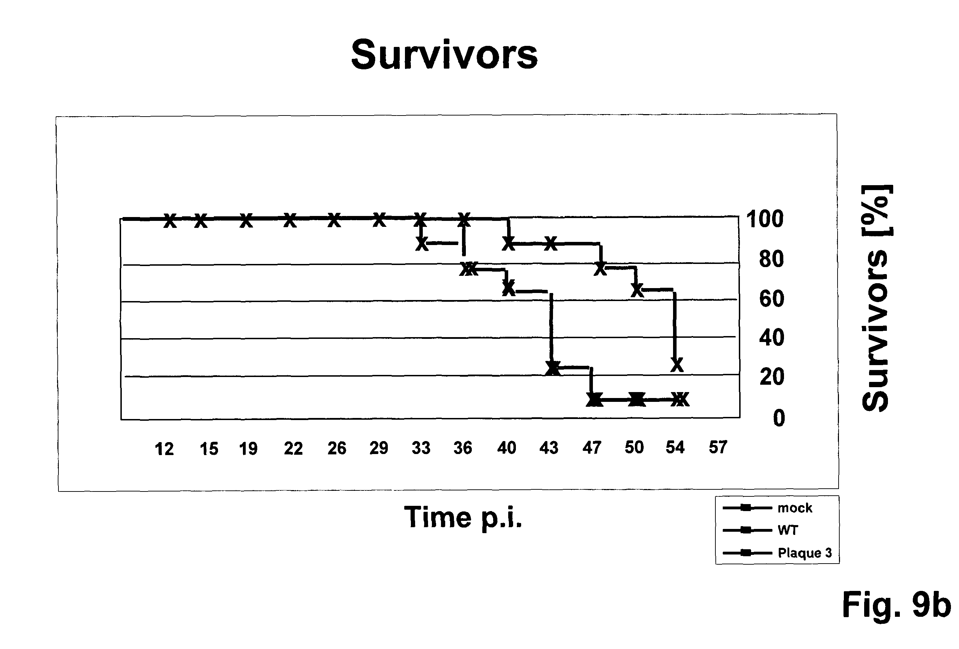

### Figure 9: Impact of H1-PV infection (wildtype and plaque #3), respectively on tumour growth of U87 glioblastoma cells subcutaneously implanted into nude balb/c mice.

### Detailed Description of the present Invention

The present invention provides a rat parvovirus H-1 variant capable of propagating and spreading through human tumour cells, characterized in that the viral polypeptides comprise the amino acid sequences shown in Figure 8B and which have a deletion from position 587 to 629 in the C-terminus of NS1 and the substitutions P124Q and E280K in NS1, a deletion from position 95 to 136 in the middle exon of NS2 and the amino acid substitution H374Y in VP1/VP2.

The term "propagating" as used herein means that the progeny virions of the parvovirus variant show amplification significantly over input levels (i.e. > 5 fold).

The term "spreading through" as used herein means that the progeny virions of the parvovirus variant are capable not only of killing primarily infected cell populations, but are able to cause secondary infections of naive cells with particles produced during the first round of infection.

The term "semi-permissive" as used herein for characterizing the host cells means that the infection with the virus results in incomplete viral replication within the particular type of host cells but are characterized by viral DNA amplification and eventually low yields of progeny virions. The latter do not necessarily have to be infectious in the beginning of the procedure.

The person skilled in the art can arrive at the parvovirus H-1 variant according to the present invention by subjecting a starting strain to serial passaging as outlined in the Examples, below, or by introducing a deletion resulting in the desired modification of the biological properties, i.e., capability of the parvovirus variant to propagate and spread through, thereby killing, human tumour cells starting from the known nucleic acid sequence and amino acid sequences of the non-structural proteins of parvovirus H-1 (Rhode and Paradiso 1983; Qiu et al., 2006) The person skilled in the art can also easily assay whether a particular variant exhibits the desired biological properties recited by using the assays explained in the examples, below.

The parvovirus variant of the invention is derived from parvovirus H-1 (H-1PV).

In principle, any tumour can be cured using a parvovirus H-1 variant of the present invention with gliomas being preferred. A particular preferred glioma is a glioblastoma.

A preferred human semi-permissive tumour cell line is NCH149. As regards the procedure of serial passaging, the person skilled in the art can easily determine how many passages are sufficient for arriving at a parvovirus H-1 variant of the present invention. In general, however, this method comprises at least 10 passages, preferably at least 20 to 25 passages, or more. Preferably, passaging is on NCH149 cells.

In order to ensure the purity and stability of the virus variants the individuals clones can be plaque-purified after passaging, preferably on NB 324K cells.

A further subject matter of the present invention relates to a nucleic acid, particularly a DNA, which codes for an above parvovirus variant.

A DNA according to the invention can be present in a vector and expression vector, respectively. A person skilled in the art is familiar with examples thereof. In the case of an expression vector for E. coli these are e.g. pGEMEX, pUC derivatives, pGEX-2T, pET3b, T7 based expression vectors and pQE-8. For the expression in yeast, e.g. pY100 and Ycpad1 have to be mentioned while e.g. pKCR, pEFBOS, cDM8, pMSCND, and pCEV4 have to be indicated for the expression in animal cells. The baculovirus expression vector pAcSGHisNT-A is especially suitable for the expression in insect cells.

In a preferred embodiment, the vector containing the DNA according to the invention is a virus, e.g. an adenovirus, vaccinia virus, an AAV virus or a parvovirus, such as MVM or H-1, a parvovirus being preferred. The vector may also be a retrovirus, such as MoMULV, MoMuLV, HaMuSV, MuMTV, RSV or GaLV.

For constructing expression vectors which contain the DNA according to the invention, it is possible to use general methods known in the art. These methods include e.g. in vitro recombination techniques, synthetic methods and in vivo recombination methods.

Furthermore, the present invention relates to host cells which contain the above described variants or vectors. These host cells include human 293(T), NBK, rat RG2 cells, NCH149, NCH82, or U87 cells. Methods of transforming these host cells, of phenotypically selecting transformants and of expressing the DNA according to the invention by using the above described vectors are known in the art.

Thus, the present disclosure also provides a method of producing the parvovirus variant of the invention, comprising the culturing of a host cell of the invention under suitable conditions and harvesting the parvovirus variant from the cells or the medium.
Moreover, the present invention relates to antibodies which specifically recognize an above describe parvovirus variant, i.e. the polypeptide region of the parvovirus variant where the deletion is located characterizing the rodent parvovirus variant in that it is capable of propagating and spreading through human gliomas. The antibodies can be monoclonal, polyclonal or synthetic antibodies or fragments thereof, e.g. Fab, Fv or scFV fragments. Preferably monoclonal antibodies are concerned. For the production it is favorable to immunize animals - particularly rabbits or chickens for a polyclonal antibody and mice for a monoclonal antibody - with an above parvovirus variant or with fragments thereof. Further boosters of the animals can be affected with the same parvovirus variant or with fragments thereof. The polyclonal antibody can then be obtained from the animal serum and egg yolk, respectively. The monoclonal antibody can be obtained according to standard methods, reference being made particularly to the method by Köhler and Milstein (Nature 256 (1975), 495) and Galfre (Meth. Enzymol. 73 (1981), 3). In this case, mouse myeloma cells are fused with spleen cells originating from the immunized animals. Antibodies according to the invention can be used in many ways, e.g. for the immunoprecipitation of the above described parvovirus variants or for the isolation thereof. The antibodies can be bound in immunoassays in liquid phase or to a solid carrier. In this connection, the antibodies can be labeled in various ways. The person skilled in the art is familiar with suitable markers and labeling methods. Examples of immunoassays are ELISA and RIA.

A kit is also provided for the application of the present invention. This kit comprises the following:
(a) a parvovirus variant according to the invention;
(b) a DNA according to the invention, e.g. an expression vector, particularly a parvovirus;
(c) an antibody according to the invention; and/or, optionally,
(d) conventional auxiliary agents, such as solvents, buffers, carriers, markers and controls.

Of component (a) to (d) one or more representatives can be present each.

The present invention also provides a pharmaceutical composition containing a parvovirus variant of the invention, vector or a cell producing said parvovirus variant ("parvotherapeutic agent" or "parvotherapeutic composition"), e.g. human 293(T), NBK or rat RG2 cells.

For administration, the parvotherapeutic agent can be combined with suitable pharmaceutical carriers. Suitable pharmaceutical carriers of a type well known in the art and readily commercially available, include phosphate buffered saline (PBS) solutions, water, emulsions such as oil/water emulsions, wetting agents of various types, sterile solutions, etc. Such carriers can be formulated with the parvotherapeutic agent(s) by conventional formulating methods for administration to the subject at a suitable dose.

Additional pharmaceutically compatible carriers can include gels, biosorbable matrix materials, implantation elements containing the therapeutic agent, or any other suitable vehicle, delivery or dispensing means or material(s). Administration of the parvotherapeutic pharmaceutical compositions to a patient, e.g. a brain tumor patient, may be effected in any of numerous suitable ways, e.g., by intravenous, intraperetoneal, subcutaneous, intramuscular, topical, intradermal, intracranial, and intratumoral administration. The route of administration, of course, depends on the nature of the disease and the specific therapeutic agent(s) contained in the pharmaceutical composition.

If such parvotherapeutic agent(s) comprise infectious virus particles with the ability to penetrate through the blood-brain barrier, treatment can be performed or at least initiated by intravenous injection of the viral therapeutic agent, e.g., a H1-PV variant.

Since long-term intravenous treatment is susceptible to becoming inefficient as a result of the formation of neutralizing antibodies to the viral therapeutic agent, different modes of administration can be adopted after an initial regimen of intravenous viral administration, or such different administration techniques, e.g., intracranial or intratumoral virus administration, can be alternatively used throughout the entire course of parvoviral treatment.

As another specific administration technique, the parvotherapeutic agent (virus, vector and/or cell agent) can be administered to the patient from a source implanted in the patient. For example, a catheter, e.g., of silicone or other biocompatible material, can be connected to a small subcutaneous reservoir (Rickham reservoir) installed in the patient during tumor removal or by a separate procedure, to permit the parvotherapeutic composition to be injected locally at various times without further surgical intervention. The parvovirus variant or derived vectors can also be injected, e.g., into a tumor, by stereotactic surgical techniques or by neuronavigation targeting techniques.

Administration of the parvoviral agents or compositions can also be performed by continuous infusion of viral particles or fluids containing viral particles through implanted catheters at low flow rates using suitable pump systems, e.g., peristaltic infusion pumps or convection enhanced delivery (CED) pumps.

A yet another method of administration of the parvotherapeutic composition is from an implanted device constructed and arranged to dispense the parvotherapeutic agent to the desired locus, e.g., tumor. For example, wafers can be employed that have been impregnated with the parvotherapeutic composition, e.g., a parvovirus H-1 variant, wherein the wafer is attached to the edges of the resection cavity at the conclusion of surgical tumor removal. Multiple wafers can be employed in such therapeutic intervention.

Cells that actively produce the parvotherapeutic agent, e.g., a parvovirus H-1 variant, or H-1 variant vectors, can be injected into the desired tumor, or into a tumoral cavity after tumor removal.

Combinations of two or more of the above-described administration modes can be employed in any suitable manner, e.g., concurrently, contemporaneously, or sequentially.

The dosage regimen of the parvotherapeutic agent is readily determinable within the skill of the art, by the attending physician based on patient data, observations and other clinical factors, including for example the patient's size, body surface area, age, sex, the particular virus, vector, cell, etc. to be administered, the time and route of administration, the tumor type and characteristics, general health of the patient, and other drugs or therapies to which the patient is being subjected.

Accordingly, the present invention also relates to the use of a parvovirus variant according to the present invention, a cell producing said parvovirus variant, a DNA, an expression vector or antibody of the present invention for the preparation of a pharmaceutical composition for the treatment of cancer. Brain tumors (preferably a glioma, medulloblastoma, meningioma or glioblastoma) are expected to be particularly amenable to treatment with an agent of the present invention.

The below examples explain the invention in more detail.

### Example 1

### Adaptation of rat H1-PV (pSR19) to human glioma

Infection of many human glioblastoma cell lines with H1-PV derived from pSR19 isolate (Accession No: NC_001358) led to the expression of viral proteins and in consequence cell killing irrespective of their resistance towards drug-induced apoptosis. However, monitoring viral DNA amplification and progeny particle production, with exception of NCH89, the majority of the tested human glioblastoma cell lines only allowed little DNA amplification and virtually no increase in progeny production (Herrero y Calle et al., 2004; DiPiazza et al., 2007). To mend this defect, it was aimed to obtain (a) propagation competent H1-PV variant(s) through serial passaging of pSR19 in human glioblastoma cells, which were competent for DNA amplification although progeny virion production was hampered. The infectious clone DNA of H1-PV, pSR19 was transfected into NCH149 cells, a cell line that was shown to allow low levels of DNA amplification by Southern blotting (Fig. 1B). Parvoviruses are dependent on (rapidly) growing cells, since conversion of the single-stranded genome to a double-stranded transcription template requires S-phase. Therefore, the initial inoculum (tranfected cells) was first amplified by dilutions of 1:4 after confluency on the surface. After reaching 4x10⁸ cells, ¾ of the cells were harvested, progeny virions were released by repeated freezing and thawing into the supernatant, and a combination of naive and transfected cells was re-infected with these virions until confluency or CPE was obtained. This procedure was continued until passage 20-25.

### Example 2

### Analyses of human glioma adapted H1-PV

After more than 20 consecutive passages the inventors obtained a virus stock competent for viral DNA amplification, progeny virion production and spreading in NCH149 cultures. The properties of this virus pool were analyzed. As shown in Fig. 1C by Southern blotting, this virus pool was characterized by strong DNA amplification after infection of NCH149 cells, producing monomeric and dimeric replication intermediates as well as single-stranded virion DNA. Besides reflecting the production of genomic viral DNA, this latter form indicates the formation of progeny virions, since the generation of single-stranded DNA is associated with the packaging process. Moreover, when supernatants of infected cells were collected (Inf 1) to infect naive NCH149 cells (Inf 2) similar DNA amplification and generation of single-stranded virion DNA was observed, demonstrating production and release of large numbers of infectious progeny virions in NCH149 cells. Indeed, release of progeny virions into the medium supernatants is dependent on the activity of cellular gelsolin, since application of neutralizing gelsolin antibodies inhibited this process. This dependency on gelsolin for the release of progeny virions indicates the involvement of an active vesicular transport to the cell periphery of progeny virions (Bar et al., 2008).

As shown in Fig. 2A by Southern blotting of the single-stranded progeny virion DNA after biochemical fractionations progeny H1-PV variant particles are found being strongly associated with vesicular fractions of infected cells. Moreover, by comparing vesicles from non-infected versus infected NCH149 cells analyzing the protein content by silver-staining a number of proteins being associated with virion-containing vesicular fractions, while being absent in non-infected cells was identified. The nature of these proteins is currently determined by MS/MS analyses.

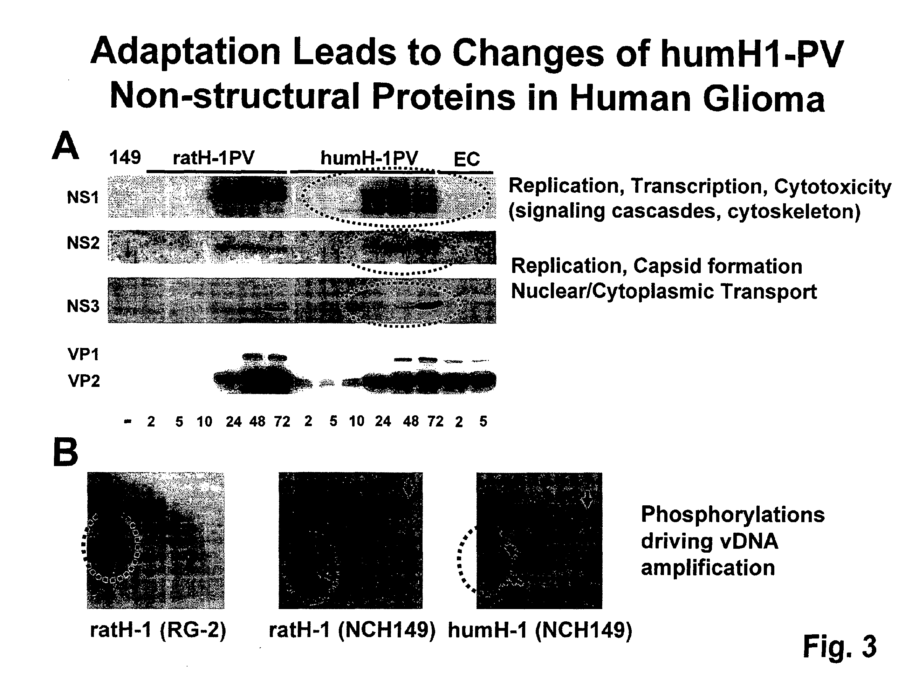

Then, the nature of the virus modification(s) causing the permissive phenotype in NCH149 cells was identified. Original H1-PV (pSR19) was produced in rat RG-2 glioma, whereas the adapted H1-PV variant (hgH1-PV) was generated in NCH149. Both virus stocks were titrated by plaque assays in permissive NB324K cells. Infections were performed at a multiplicity of 10 in NCH149 cells and the production of viral proteins was determined in a time-course experiment by Western blotting. As shown in Fig. 3A, remarkable differences in the expression levels of the small non-structural proteins NS2 and NS3 were observed, while NS1 from hgH1-PV proved to be by far more heterogenous than NS1 from pSR19. NS1 is a multifunctional phosphoprotein, which is required for many processes during virus amplifications and is regulated for its functioning by differential phosphorylations. Indeed, as shown in Fig. 3B, when the phosphorylation patterns of NS1 obtained from pSR19 after infections of rat RG-2 cells and human NCH149 cells was analyzed the lack of two characteristic peptides after infection of NCH149 (encircled) was observed. In contrast, hgH1-PV was able to generate these phosphopeptides (and an additional peptide marked with an arrow), accounting for the differences in the migration pattern observed by Western blotting. Moreover, detailed functional analyses of the MVM NS1 phosphorylation (pattern), which closely resembles the one observed for pSR19 in RG-2/hgH1-PV in NCH149 suggests that the two encircled peptides are important phosphorylations involved in the replicative functions of the NS1 protein.

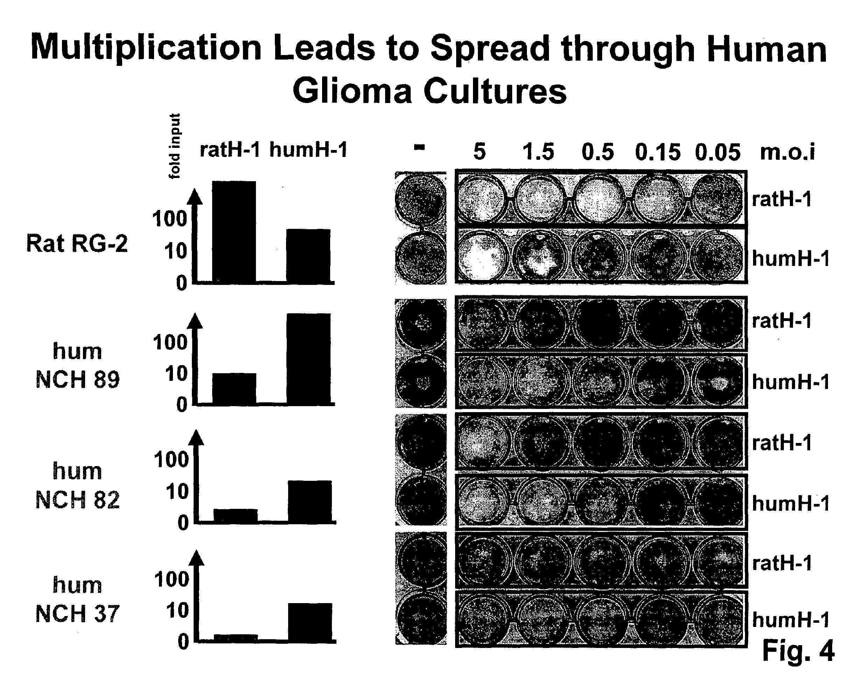

Finally, the adapted hgH1-PV stock was tested for its fitness to propagate and spread through a variety of (human) glioblastoma cultures in comparison to the pSR19 isolate. As shown in Fig. 4. in addition to NCH149 cells, all other human glioblastoma cell lines tested so far proved to be proficient to amplify hgH1-PV (but not pSR19) progeny virions at least ten fold above input levels and proved superior to pSR19 to spread through the cultures.

### Example 3

### Genetic characterization of human glioma adapted H1-PV

To determine genetic variations hgH1-PV acquired during passaging in NCH149 as compared to the original inoculum (pSR19), endpoint titrations were performed to plaque purify individual virus variants and virion DNA was isolated. As shown in Figs. 5 and 5b, DNA of six individual plaques was obtained, the whole coding region was amplified by PCR in three steps and the overlapping fragments were cloned into pCR2.1. Genetic variations were determined by sequencing and compared to the published sequence of pSR19 (Accession No NC_001358). Mutations as compared to the pSR19 sequence found to be present on the individual plaque isolates are indicated, numbered and positioned in the H1-map. The detailed sequence alterations are shown in Figs. 6 and 6b.

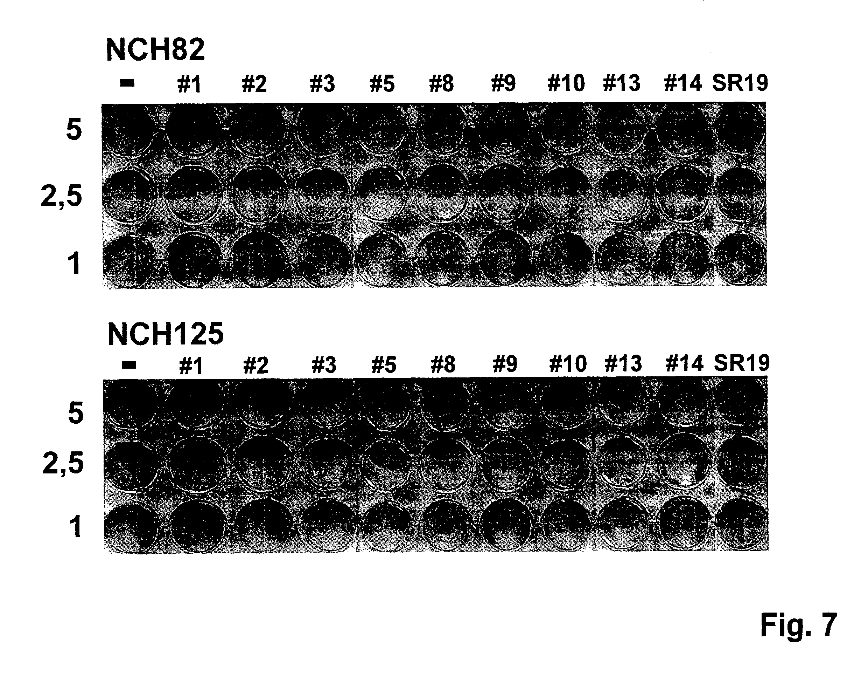

To further characterize the properties of individual clones, virus stock of individual plaque isolates was generated and their fitness to spread and kill human glioblastoma cell cultures in comparison to pSR19 generated in rat RG-2 cells was tested. As shown in Fig. 7, all isolates harbouring a deletion mutant in the C-terminal part of NS1/middle exon of NS2 surpassed pSR19 in their fitness to spread and kill human glioblastoma cells, confirming the initial findings with the pooled variants and demonstrating that the deletion mutant is responsible for the observed host range switch.

### Example 4

### Impact of H1-PV infection (wildtype and plaque #3) on tumor growth

U87 glioblastoma cells were infected or not *ex vivo* with a MOI of 1,5 pfu/cell. (WT:H-1PV wildtype; Plaque #3: adapted H-1PV derived from plaque #3, mock: uninfected U87 cells). Cells were washed and dissolved in PBS 4 h p.i. and 2 X 10⁶ mock or H-1PV infected cells in 100 µl PBS were implanted subcutaneously into 8 mice per group. Tumour size (length, width, height) was measured mechanically with a calliper twice a week, starting 12 days after tumour cell implantation. Volume was calculated as follows: ½ x length x width². Animals were sacrificed at a tumour size of maximum 2500 mm³, in case of appearance of peritoneal carcinosis or malignant ascites. All surviving animals were sacrificed at day 61 after cell implantation. (1 X WT, 2 X Plaque #3) as indicated by "survivors". It was shown in Figs. 9, 9a and 9b that the tumor inhibition of plaque #3 was stronger than with H-1PV wild type. Furthermore, plaque #3 led to more survivors than H-1PV wild type.

### List of References

Rhode, S.L. III and Paradiso, P.R. (1983. Parvovirus genome: nucleotide sequence of H-1 and mapping of its genes by hybrid-arrested translation. J. Virol. 45, 173-184.
Qiu, J., Yoto, Y., Tullis, G., and Pintel, D.J. (2006). Parvovirus processing strategies. pp.253-274. In "Parvoviruses" Eds. Kerr, J.R., Cotmore, S.F., Bloom, M.E., Linden, R.M., and Parrish, C.R. Hodder Arnold, London.

## Claims

1. A rat parvovirus H-1 variant capable of propagating and spreading through human tumour cells, **characterized in that** the viral polypeptides comprise the amino acid sequences shown in Figure 8B and which have a deletion from position 587 to 629 in the C-terminus of NS1 and the substitutions P124Q and E280K in NS1, a deletion from position 95 to 136 in the middle exon of NS2 and the amino acid substitution H374Y in VP1/VP2.

2. The rat parvovirus H-1 variant of claim 1, wherein the tumour cells are glioma cells.

3. The rat parvovorius H-1 variant of claim 2 wherein the glioma is a glioblastoma.

4. A DNA, coding for the rat parvovirus H-1 variant according to any one of claims 1 to 3.

5. An expression vector, comprising the DNA according to claim 4.

6. A host cell containing the rat parvovirus H-1 variant of any one of claims 1 to 3 or the expression vector according to claim 5.

7. An antibody, directed against the NS1 protein of a parvovirus variant according to any one of claims 1 to 3 further **characterized in that** it does only bind to the variant protein having the deletion but not to the wild type protein.

8. Kit comprising:
(a) a rat parvovirus H-1 variant of any one of claims 1 to 3;
(b) a DNA according to claim 4;
(c) an antibody according to claim 7; and/or, optionally,
(d) conventional auxiliary agents, such as solvents, buffers, carriers, markers and controls,
wherein of components (a) to (d) one or more representatives can be present each.

9. A pharmaceutical composition containing (a) a rat parvovirus H-1 variant according to any one of claims 1 to 3, the DNA of claim 4, the expression vector of claim 5, the host cell of claim 6 and (b) a pharmaceutically acceptable carrier.

10. Use of a rat parvovirus H-1 variant according to any one of claims 1 to 3, the DNA of claim 4, the expression vector of claim 5 or the host cell of claim 6 for the preparation of a pharmaceutical composition for the treatment of cancer.

11. The rat parvovirus H-1 variant according to any one of claims 1 to 3, the DNA of claim 4, the expression vector of claim 5 or the host cell of claim 6 for use in a method of treating cancer.

12. The use of claim 10 or 11, wherein said cancer is a brain tumor.

13. The use of claim 12, wherein said brain tumor is a glioma, medulloblastoma, meningioma or glioblastoma.

## Patentansprüche

1. Ratten-Parvovirus-H-1-Variante, die sich in menschlichen Tumorzellen vermehren und ausbreiten kann, **dadurch gekennzeichnet, dass** die viralen Polypeptide die in Figur 8B gezeigten Aminosäuresequenzen umfassen, die eine Deletion von Position 587 bis 629 im C-Terminus von NSI und die Substitutionen P 124Q und E280K in NS1, eine Deletion von Position 95 bis 136 im mittleren Exon von NS2 und die Aminosäuresubstitution H374Y in VP1/VP2 aufweisen.

2. Ratten-Parvovirus-H-1-Variante nach Anspruch 1, wobei die Tumorzellen Gliomzellen sind.

3. Ratten-Parvovirus-H-1-Variante nach Anspruch 2, wobei das Gliom ein Glioblastom ist.

4. DNA, die für die Ratten-Parvovirus-H-1-Variante nach einem der Ansprüche 1 bis 3 kodiert.

5. Expressionsvektor, umfassend die DNA nach Anspruch 4.

6. Wirtszelle, enthaltend die Ratten-Parvovirus-H-1-Variante nach einem der Ansprüche 1 bis 3 oder den Expressionsvektor nach Anspruch 5.

7. Antikörper, der gegen das NS1-Protein einer Parvovirus-Variante nach einem der Ansprüche 1 bis 3 gerichtet ist, weiter **dadurch gekennzeichnet, dass** es nur an das die Deletion aufweisende Varianten-Protein nicht aber an das Wildtyp-Protein bindet.

8. Kit, umfassend:
(a) eine Ratten-Parvovirus-H-1-Variante nach einem der Ansprüche 1 bis 3;
(b) eine DNA nach Anspruch 4;
(c) einen Antikörper nach Anspruch 7; und/oder optional
(d) herkömmliche Hilfsmittel, wie beispielsweise Lösungsmittel, Puffer, Träger, Marker und Kontrollen,
wobei von den Komponenten (a) bis (d) jeweils einer oder mehrere Vertreter vorhanden sein können.

9. Pharmazeutische Zusammensetzung, enthaltend (a) eine Ratten-Parvorvirus-H-1-Variante nach einem der Ansprüche 1 bis 3, die DNA nach Anspruch 4, den Expressionsvektor nach Anspruch 5, die Wirtszelle nach Anspruch 6 und (b) einen pharmazeutisch annehmbaren Träger.

10. Verwendung einer Ratten-Parvovirus-H-1-Variante nach einem der Ansprüche 1 bis 3, der DNA nach Anspruch 4, des Expressionsvektors nach Anspruch 5 oder der Wirtszelle nach Anspruch 6 zur Herstellung einer pharmazeutischen Zusammensetzung zur Behandlung von Krebs.

11. Ratten-Parvovirus-H-1-Variante nach einem der Ansprüche 1 bis 3, DNA nach Anspruch 4, Expressionsvektor nach Anspruch 5 oder Wirtszelle nach Anspruch 6 zur Verwendung in einem Verfahren zur Behandlung von Krebs.

12. Verwendung nach Anspruch 10 oder 11, wobei der Krebs ein Hirntumor ist.

13. Verwendung nach Anspruch 12, wobei der Hirntumor ein Gliom, Medulloblastom, Meningiom oder Glioblastom ist.

## Revendications

1. Variante de parvovirus de rat H-1 à même de se propager et de se répandre dans les cellules tumorales humaines, **caractérisée en ce que** les polypeptides viraux comprennent les séquences d'acides aminés représentées à la figure 8B et qui présentent une suppression de la position 587 à la position 629 dans l'extrémité terminale C de NS1 et les substitutions P124Q et E280K dans NS1, une suppression de la position 95 à la position 136 dans l'exon central de NS2 et la substitution d'acide aminé H374Y dans VP1/VP2.

2. Variante de parvovirus de rat H-1 selon la revendication 1, dans laquelle les cellules tumorales sont des cellules de gliome.

3. Variante de parvovirus de rat H-1 selon la revendication 2, dans lequel le gliome est un glioblastome.

4. ADN codant la variante de parvovirus de rat H-1 selon l'une quelconque des revendications 1 à 3.

5. Vecteur d'expression, comprenant l'ADN selon la revendication 4.

6. Cellule hôte contenant la variante de parvovirus de rat H-1 selon l'une quelconque des revendications 1 à 3 ou le vecteur d'expression selon la revendication 5.

7. Anticorps, dirigé contre la protéine NS1 d'une variante de parvovirus selon l'une quelconque des revendications 1 à 3, **caractérisé par** ailleurs en ce qu'il se lie uniquement à la variante de protéine présentant la suppression, mais pas à la protéine de type sauvage.

8. Kit, comprenant:
(a) une variante de parvovirus de rat H-1 selon l'une quelconque des revendications 1 à 3;
(b) un ADN selon la revendication 4;
(c) un anticorps selon la revendication 7; et/ ou, optionnellement,
(d) des agents auxiliaires conventionnels, tels que des solvants, des tampons, des supports, des marqueurs et des contrôles, dans lequel, des composants (a) à (d), un ou plusieurs représentants peuvent, chacun, être présents.

9. Composition pharmaceutique contenant (a) une variante de parvovirus de rat H-1 selon l'une quelconque des revendications 1 à 3, l'ADN selon la revendication 4, le vecteur d'expression selon la revendication 5, la cellule hôte selon la revendication 6 et (b) un support pharmaceutiquement acceptable.

10. Utilisation d'une variante de parvovirus de rat H-1 selon l'une quelconque des revendications 1 à 3, de l'ADN selon la revendication 4, du vecteur d'expression selon la revendication 5 ou de la cellule hôte selon la revendication 6 pour la préparation d'une composition pharmaceutique pour le traitement du cancer.

11. Variante de parvovirus de rat H-1 selon l'une quelconque des revendications 1 à 3, l'ADN selon la revendication 4, le vecteur d'expression selon la revendication 5 ou la cellule hôte de la revendication 6 destinés à être utilisés dans un procédé de traitement du cancer.

12. Utilisation selon la revendication 10 ou 11, dans laquelle ledit cancer est une tumeur cérébrale.

13. Utilisation selon la revendication 12, dans laquelle ladite tumeur cérébrale est un gliome, un médulloblastome, un méningiome ou un glioblastome.
